# EUROPEAN PATENT APPLICATION

(11) **EP 2 380 973 A1**
(43) Date of publication of application: **26.10.2011**
(21) Application number: 09836446.6
(22) Date of filing: 20.08.2009
(51) Int. Cl.: C12N 5/095, C12N 5/10, A61K 9/51, A61P 35/00

(54) **DRUG MADE FROM STEM CELLS WITH REPROGRAMMED CELL SIGNALING, METHOD FOR PRODUCING SAID PREPARATION AND THE USE THEREOF**

(30) Priority: 30.12.2008 RU 2008152343
(71) Applicant: Bryukhovetskiy, Andrey Stepanovich, Moscow 117437 (RU)
(72) Inventor: SEVASTIANOV, Viktor Ivanovich, Moscow 125480 (RU)
(74) Representative: Boeckh, Tobias
(86) International application number: PCT/RU2009/000424
(87) International publication number: WO 2010/077168

(57) **Abstract**

The invention is applied in the therapy of brain and spinal cord tumors, degenerative, hypoxic, ischemic diseases and traumatic injuries of the central nervous system (CNS) and other diseases of humans and animals. Object of invention is to provide target delivery of signaling substance to the pathological area of an organism, triggering in due time and long-time support of active signaling intercellular action of specific therapeutic character of a healthy and well controlled stem cell (SC) onto controlling system of defective cells. The preparation of SC with reprogrammed cell signaling comprises the basic preparation of SC, the membrane, and/or nucleus, and/or cytoplasm of which contains implanted protein or pharmaceutical able to regulate signaling pathways of SC and cells of pathological focus in a mammal organism, provisionally encapsulated in nanocontainers of less than 100nm size, obtained from biodegradable material, intact for organelles and compartments of SC of the basic preparation. The material has a set biodegradation period in a mammal organism to provide programmed exit of protein or pharmaceutical in intra-or intercellular space thus reprogramming signal transduction of key genes in the desired therapeutic orientation of physiologic events in the cell cycle directly in the pathological area or tissue of an organism.

## Description

### Field of the Invention

The invention belongs to the field of medicine, namely, neurology, neurosurgery and oncology, and is designed to treat the tumors of brain and spinal cord, degenerative, hypoxic, ischemic diseases and traumatic consequences of central nervous system (CNS) injuries and other disorders of humans and animals.

### Description of Related Art

The challenge of the therapy of tumors, degenerative diseases and brain and spinal cord injuries remains extremely difficult and practically unsolved. Despite considerable advance of modern life medicine, survival rate in the case of brain glial tumor varies from 12 to 15 months. No significant results have been obtained in the therapy of degenerative, hypoxic and ischemic disorders of neural system. This seems to be associated with limited regeneration and sanogenesis capacity of neural tissue as well as characteristics of neurogenesis in adult CNS on the one hand, and specific features of pathogenesis of certain nervous disorders.

However, past two decades have changed understanding of available opportunities to treat the above mentioned disorders, which is underlain by accumulation of scientific evidence on regenerative potential of CNS, discovery of the accumulation mechanism of genetic mutations in neural cells of brain and spinal cord in case of CNS pathology and irrefutable proofs of the possibility to control the processes of regeneration, angiogenesis and synaptogenesis in nervous tissue ([1], c. 167, 194).

The concept of a cancer (mutant) stem cell, discussed in current scientific literature has opened new horizons in the treatment of oncological diseases and allows for new perception of pathogenetic basis of neurooncological disorders and certain neurological ones [2]. The series of discoveries in the end of 20-th century and beginning of 21-st in cell and molecular biology about regulation of cell events in a neural cell (differentiation, apoptosis, proliferation etc) offered a different view on the pathogenesis of a whole range of neural disorders. Key mechanisms suppressing tumor genes and signaling processes in a nervous cell have been disclosed, the patterns of disordered cell regulation signaling system under pathological brain condition have been determined [3]. Development of new methods of production to obtain wide range of chemicals from signaling proteins of cellular and intercellular regulation and understanding of basic mechanisms of their action led to unique experimental results of their application in animals and humans that opened new prospects for the therapy of oncological and neural disorders. Development and application of new medications based on intra- and extracellular signaling proteins permitted significant progress in the therapy of previously incurable oncological diseases. Direct action of signaling proteins onto cancer and healthy stem cells have been proved. However, enthusiasm about clinical application of signaling cell enzymes, proteins and antibodies to them to treat neurooncological and certain neurologic disorders was replaced by disappointment and discontent with the obtained results. Numerous research of the medications based on signaling proteins showed that therapeutic concentrations of these proteins were harmful for intact neural tissue of humans thus significantly reducing the opportunity to apply them in clinical practice [4].

Recent 15 years the hope for breakthrough in the therapy of neurologic and neurooncological disorders was laid on clinical application of stem cells (SC) [5]. Discovery of neural stem cells in adult brain developed new understanding of the essence of neurogenesis [6]. Still, success of empiric and experimental application of cell therapy for neurological diseases is very humble and clinical use of SC is limited and has a whole range of significant disadvantages ([1], c. 360).

In the late 90-s accumulation of new data about the character and structure of genetic defects in the case of some brain tumors and neurological diseases provided the basis for new science, namely gene therapy that also held much promise [4]. Unfortunately the achievements of gene engineering and gene therapy are quite limited and so far have not been crowned with success, notwithstanding broad opportunities it offered to neurology, neurosurgery and neurooncology.

Therefore the question appears, why breakthrough in basic sciences, cell and molecular biology, did not entail biotechnological breakthrough in clinical research? The answer seems simple, but controversial. From the perspective of information approach ([1], c. 37-58) these controversies can be explained by mismatch of the advances and available approaches received at different information levels of scientific research. Technological breakthrough in biology took place at subcellular, molecular, atomic levels and extrapolation of its result onto the level of tissue and organ seems complex and of little promise. This is the reason of the failure to apply modern recombinant proteins of cell signaling of cytokine and neurotrophin families and information molecules of signaling proteins of inter- and intracellular interaction in the clinical practice. It should be stressed that molecules of signaling proteins and their antibodies are quite unstable and function in micro- and nano-range. Efficiency of their application in system (oral, parentheral, intracthecal etc.) administration is considerably limited by abrupt fall of their concentration in biological fluids of a body when dissolved in blood, CSF, lymph. Moreover, molecules of signaling protein have very short active period of their information effects along with large opportunities to affect various cells of somatic organs while circulating in biological fluids [7]. Extremely low concentrations of signaling proteins in the lesion sites of brain compel the researchers to increase therapeutic concentration of the medication in blood to the value toxic for healthy neurons, brain vessels and somatic organs. Consequently total and uncontrollable misbalance of signaling protein action is observed entailing regulatory misbalance of cell functioning of the organs accompanied by severe complications from such therapy [4].

There are works that describe basic approaches of complementation gene therapy of tumors and infection diseases with fully active gene. Thus, development of autologous modified T-lymphocytes using retroviruses with installed genes allows for successful application of gene constructions to correct immune deficiency [4]. Cloned gene or artificial molecules of RNA or DNA function as a pharmaceutical in such gene therapy. Most frequently, these researches transduce gene in the cells with retrovirus (lentivirus) bound with certain gene.

The technology to use astrocytes in order to induce apoptosis of malignant glioma is the closest analogue (prototype) of the present invention (Uzzaman M, Keller G., Germano Isabelle M. In vivo Gene Delivery by embryonic stem cell-derived astrocytes for malignant gliomas. Departments of Neurosurgery (MU, IMG) and Gene and Cell Medicine (GK), Mt. Sinai School of Medicine, New York, USA. Neuro Oncol Advance Publication DOI:.1215/15228517-2008-056, August 1, 2008). This research demonstrates that astrocytes obtained from embryonic stem cells (ESC) under certain conditions express genes that can be used to induce apoptosis of malignant glioma cells in vitro. Gene TRIAL (TNF-dependant ligand inducing apoptosis) initiates apoptosis in various tumor cells, including those of glioma. The authors evaluated apoptotic effect of transgenic TRIAL application delivered by astrocytes from embryonic SC, in malignant gliomas in vivo. The cells of human malignant glioma A172 that formed heterotopic xenotransplant were transplanted to nude mice, and then, embryonic SC astrocytes producing TRIAL were implanted into the transplants. TRIAL expression in xenotransplants of malignant glioma was confirmed by PCR diagnostics with reverse transcription and by immunohistochemical test after external induction of the gene. 48 hours post astrocytes injection experimental group demonstrated significant reduction of tumor size - 14% after single dose and 31% after double dose. TUNEL staining demonstrated large number of apoptotic tumor cells. Seven days post injection the tumor was subject to necrosis. Expression of death receptor (DR4) was much higher in experimental groups as compared to controls. The authors of the work suppose that astrocytes obtained from ESC and producing RAIL can be used as the vectors to deliver genes to malignant gliomas.

However, it should be noted that the above mentioned technical solution, as well as many other methods of gene therapy have significant limitations and to date have not been introduced into clinical practice. Application of recombinant viral vector is extremely harmful and according to the data, provided by Kiselev V.I [4], has once led to the death of a 18-year old patient during the I phase of clinical trial. Lethal outcome from respiratory failure, kidney failure and cortical necrosis resulted from administration of genetically engineered construction. Hence, the authors of the present invention completely refuse from transgenic constructions of viral plasmids, retroviruses and lentiviruses, bound with the genes for their transduction in pathological cells.

### Brief Summary of the Invention

Currently inflammation and stress factor is proved to trigger pathological mechanisms in neural cells in the most of the cases of neurologic diseases, while disordered mechanisms of cell regulation with the exact location, i.e. genetic disorder in the nuclei of neural cells, are dominating processes in the pathological neural tissue. Most of neurological manuals describe typical genetic mutations detected in the cells in the cases of brain tumors, Parkinson's disease, Alzheimer's disease, senile dementias, amyotrophic lateral sclerosis, encephalopathies of vascular and toxic genesis and of a whole range of degenerative atrophic disorders of CNS. To date modern methods of genome and proteome analysis of the cells of lesion permit easy detection of such genetic disorders. Interpretation of these advanced tests is the main challenge, along with the inability to correct detected genetic defects. Gene therapy to correct concrete molecular disorder is methodologically justified, but accompanied by many a technical and technological problem of modern stage of scientific research. Solution can be offered by setting a new vector (direction) to the development of a cell cycle of a pathological cell and the cells of a pathological area, and not by correction of exact genetic modification of mutant genes (genetic defect) and replacement of damaged gene. In this case, considering cluster type of the involvement of cell genes into biocontrol and regulation, gene mutations (even in several locuses of chromosomes) leading to certain cell cycle will not be determinant for life and development of a whole cell. For example, if the outcome of pathogenetic processes in neural tissue has triggered intracellular apoptosis of mutant neural SC, then it is obvious that the vector of all cell processes should be changed to induce differentiation or proliferation or vice versa. This can be achieved through a new approach of reprogrammed transduction (expression or inhibiting) of key genes with signaling proteins. Function of a key gene of tumor suppression p53 provides illustration to it. Downregulation of the gene initiates the whole cascade of genes in a cell nucleus resulting in anti-apoptotic effect, neoangiogenesis, proliferation and cell proliferation (pathogenetic and pathophysiologic conditions for tumor development), while upregulation of p53 initiates apoptosis, hinders angiogenesis, cell migration and blocks formation of new cells (pathogenetic components of atrophic and degenerative processes in brain). Hence, control of key genes can serve as basis for the control of main cell processes in the researcher determined (reprogrammed) orientation of the development of certain physiologic and algorithmised processes in a cell.

Consequently, considering insufficient biotechnological basis of gene therapy and absence of adequate gene carrier, it is practical to refuse from disintegration and arrest of the functioning of pathological neural cells through correction of the defects of certain mutant genes, and to attempt to biologically correct cell processes in the pathological tissue, setting programmed vector to orient cell cycle development. To date all theoretical knowledge and technical opportunities are available for such biological correction (biological regulation). The mechanisms of cellular and intercellular signaling, development of apoptosis, cell differentiation, proliferation and other cell processes are well studied. The large number of signaling proteins participating in these processes are decoded and their recombinant copies and antibodies have been prepared and are currently sold by the leading biotechnological companies, Sigma-Aldrich Corp. (USA) and Millipore Corporation (CIIIA), and widely applied in clinical trials in oncology, neurology and neurosurgery by various pharmaceutical companies.

However, a ubiquitous "minibot" to deliver signaling proteins to the site of action is not available so far. For it scientific literature proposes such candidates as modified bacteria, genetically engineered constructions and self-assembling atomic nanoconstructions (nanobots). Various immune monoclonal self-orienting antibody binary systems with streptavidin and biotin and immunoliposomal nanoconstruction have been developed to transport signaling molecules to different cells of tumor or pathological site [4]. Still, the effects achieved by these agents are very rough and destructive, so they cannot be used to correct cell signaling and for biocontrol of the processes in a cell.

Therefore, the development of a new transfer mechanism became necessary in order to transfer signaling information bypassing cells of somatic organs and intact brain cells, to integrate interactions at different information levels and to establish the system of biocontrol and regulation of pathological process at cellular and intercellular levels. New methodology and new logistics of target transfer of the signals to concrete genes of target cells in CNS is requisite.

Fundamental patterns and mechanisms of regulation of intra- and extracellular biosignaling can be developed on the basis of fundamental principles of system control theory [8, 9]. For that certain algorithms of short term directing signaling action (expression, stabilization, inhibition) onto specific (key) genes of a cell must be developed, and on the basis of these algorithms signaling controlling action towards the object of control (OC) must be programmed. OC must adequately and constantly react to cell signaling. The response to OC must be monitored and registered permitting regular control and regulation of it. Power, frequency and duration of the signals must not destruct controlling system presented by a cell. Available techniques of liposomal constructions and binary systems do not answer this purpose due to very "rigid" parameters of action.

A healthy SC of a human and animals are perceived by the authors as controllable "biobot" to transfer signals to a pathological cell, as a healthy SC is a universal controlling system genetically programmed for restoration of the injuries and for regeneration in the inured organs and tissues. It has a unique ability to migrate to the site of pathology following concentration gradient of chemokines and local pro-inflammatory factors and is able to fulfill complete function of transfer for necessary signaling molecules. Analysis of a whole range of scientific evidence of SC functioning in the site of lesion demonstrates that in case of certain events of pathological cells (for example, in case of apoptosis), healthy SC receives specific signals from these cells, processes them and outputs return signals (to "survive") to the injured cells. Hence, SC perform intercellular and intracellular biocontrol of local sanogenesis and reparative functions of the cells in tissue. Depending on severity of injury pathological cells react to these signals (cascade of cytokines and neurotrophic and other information factors, produced by SC) and signaling systems promoting formation of necessary regenerative processes.

Thus, the main task of the claimed invention is to develop the stem cell preparation with reprogrammed cell signaling that would provide target delivery of signaling substances (i.e. signaling proteins and pharmaceuticals regulating signaling pathways) exactly to the site of lesion of a mammal organism, as well as initialization in due time and long-term support of active signaling intercellular action of preset therapeutic feature of the intact and well controlled SC onto the controlling system of the deficient (mutant neural or cancer) cell.

In the invention the task is solved by the suspension of stem cells with reprogrammed cell signaling that contains basic preparation of stem cells the membranes and/or nucleus and/or cytoplasm of which contains protein or pharmaceutical. The protein or pharmaceutical is able to regulate signaling pathways of stem cells and of the cells of the pathological focus in the mammal, and is encapsulated into nanocontainers made of biodegradable material that is intact to organelles and compartments of the stem cells of the basic preparation and has predetermined period of biodegradation in a mammal organism in order to provide programmed exit of protein or pharmaceutical into intra- or intercellular space of the stem cells thus reprogramming signal transduction of key genes of the stem cells in specific therapeutic direction of the development of cell cycle events directly in the site of lesion of tissue.

Autologous stem cells are used as basic preparation of stem cells.

The size of nanocontainers does not exceed 100 nm.

Nanocontainers made of biopolymer materials can be used as nanocontainers. Co-polymers polylactide glycolide and/or polyhydroxybutirate-valerate can be used as biopolymer materials.

Also nanocontainers made of liposomes or nanoemulsions can be used as nanocontainers.

The other task of the present invention is the development of the method of production of the preparation of stem cells with reprogrammed cell signaling.

The solution of the second task is achieved through the method of production of the preparation of stem cells with reprogrammed cell signaling, selection of protein or pharmaceutical, able to regulate signaling pathways of stem cells and cells of pathological focus in a mammal organism, encapsulating of selected protein or pharmaceutical in nanocontainers of biodegradable material, intact for organelles and compartments of stem cells of the basic preparation with preset period of biodegradation in a mammal organism and further implantation of nanocontainers in the membrane and/or nucleus and/or cytoplasm of the stem cells of basic preparation.

The preparation with autologous stem cells is selected as the basic preparation.

Encapsulation can be performed though ultra-dispersion of the mixture of biopolymer material with protein or pharmaceutical.

Nanocontainers can be implanted by direct intracellular injections of suspension or emulsion of nanocontainers into membrane, cytoplasm or nucleus of stem cells of basic preparation, as well as by means of endocytosis of nanocontainers into cytoplasm of the stem cells, when suspension or emulsion of nanocontainers is exposed to basic preparation.

Predominantly the claimed preparation is produced in sterile conditions or directly in an operation room (ex temporo) or in a cultural laboratory.

Moreover, according to the present invention the claimed preparation of SC is proposed to be applied for intra- and extracellular regulation of signaling pathways of pathological cells in the therapy of diseases, injuries and tumors of brain and spinal cord, as well as metastatic lesions of other organs and systems of a mammal.

The mechanism of action of the claimed preparation of SC with reprogrammed cell signaling is conditioned by targeted migration of signaling molecules of protein or pharmaceutical contained in the SC preparation and encapsulated into biodegradable containers to the pathological area of brain or other organ of a mammal. Predetermined biodegradation of nanocontainers leads to programmed release of signaling molecules from nanocontainers into SC or intercellular space of pathological area and signaling molecules affect intra- and extracellular signaling pathways and gene transduction in order to initiate therapeutic inductive cell processes (instructive apoptosis, controlled differentiation, inductive proliferation etc) in the site of lesion.

Theoretical and experimental grounds of the claimed cell preparation with reprogrammed cell signaling were provided by the authors' own research, as well as published scientific evidence [6], demonstrating that SC are able to migrate to tumor and/or site of lesion in the brain following concentration gradient of inflammatory chemokines. The evidence of migration abilities of neural stem cells (NSC) and hematopoietic stem cells (HSC) proved the migration phenomenon to be universal for all types of SC. The capacity of target migration of SC to pathological sites of the brain permits to use SC as a universal vehicle to deliver requisite information and signaling action to the sites of pathology.

The preparation was developed in experimental model of brain glioma (C6 glioma) but the development and experimental application of it disclosed universal opportunities to apply it not only to CNS disorders, but also to the disorders of other organs and systems of humans and animals.

It should be noted that as proposed in the present invention, healthy SC can be used as the transport to deliver signaling substances to the lesion site, only under specific conditions:
1. Acute inflammation in the site of lesion must be over and the action of dominant local inflammatory and destructive pathogenetic factors of injury must be reduced to minimal.
2. Injury or pathogenic influence must not lead to irreversible mutation of genes in the nuclei of the injured cells.
3. The opportunities to retain functioning microenvironment of pathological cells must be preserved.

In the rest of the cases a pathological cell becomes insensitive to controlling signals of healthy SC. Moreover, medical literature describes the cases of tumor formation after SC transplantation caused by transformation of healthy SC into mutant (cancer) SC under the influence of the factors of inflammation and necrosis. Hence, primary and even cultured SC without changing their cell signaling can hardly be applied in case of acute inflammation, autoimmune process, atrophic process, tumors and most often is contraindicated in neural diseases. In these cases it is necessary to enhance natural bioregulation and regeneration of autologous SC, which is possible only in the case of reprogramming of functional capacities of the primary SC at genetic level.

Regarding the basic principles of the system control theory, according to the claimed invention a pathologically modified mutant cell becomes the object of control (OC), and the control should be performed by the healthy controllable SC. For better understanding and prognosis of OC behavior, cybernetic principle of mathematic theory of oscillation can be involved according to which the slowest process is the controlling one [8]. Extrapolating mathematic approach to the system of cell interrelations one can say that the slowest process in the development of a cell cycle is the functioning of SC. The neural SC demonstrates the slowest activity of biological processes in neurogenesis (complete cycle of adult SC division makes 21 days, and its precursors - 12 hours). Mutant (for example cancer) SC in glioma has slower cycle of division (28 days), thus rendering healthy SC controlled and subordinate. Hence the signals of the healthy SC are not controlling for the mutant (cancer) SC. Slowness of biological processes in cancer cells makes them low-sensitive to radiation and chemical therapy, while actively dividing cells fully response to radiation and chemical action. Mutant SC are most likely to function according to the principle of open-loop control, consisting in setup unit (setup signal) rigidly determining control program. Here the control does not take into account the impact of perturbations onto parameters of the process. Healthy SC function according to the principles of compensation and feedback. Compensation principle consists in the possibility to correct controlling action if deviated from the setup value at OC input by summing up the signal of controlling action with correcting action, proportional to perturbation and compensating for its influence. The principle of feedback is based on controlling action being corrected depending on output value of a signal. In case the value of output value deviates from the required, the signal is corrected to reduce the deviation.

Hence, cybernetics provides explanation to the inefficiency of the available therapy of neurooncologic and neurodegenerative pathologies. The solution of these issues should rely on new methodologic positions. Principle of open loop control can be illustrated with the tape-recorder that functions in the set mode of audio reproduction and does not react to external actions. However, signaling action (rheostat installed into electric circuit) can slow down or speed up sound reproduction. Applying all the above said to the language of biology, specific dominant signals can enhance activity of controlling mutant SC and make it controllable in the general continuum of the examined cell systems, to subordinate to the signaling of healthy SC and, consequently to modify functioning of cell cycle for therapeutic needs. Moreover, modification of functional activity of cancer SC can make it susceptible to standard regimen of radiation and chemical therapy.

According to the above said the issue of the development of new methods to treat neural and oncologic disorders with the claimed invention modifies into the issue of control of interrelations of two biological systems, i.e. mutant (cancer) SC, and healthy SC.

Solving the abovementioned task in triggering and supporting active signal intercellular action of the healthy SC onto controlling system of defective cell, it is necessary to provide the steady signal of specific power inside a healthy stem cell, considering that the healthy SC being a genetically determinant biological object is able to migrate to the site of lesion or tumor. In this regard it is not difficult to trigger key genes affecting known signaling mechanisms. SC reprogramming can be launched through chemical, physical or interventional action of signaling recombinant proteins or their antibodies onto SC in vitro before transplantation of SC. In certain cases the effect of this action will be rather strong, but short-termed and unstable. Accordingly it can misbalance the functions of the SC compartments and disturb its migration and navigation features. Hence, the claimed invention offers novel technical solution in reprogramming of transduction of the somatic SC key genes, and namely to encapsulate proteins and pharmaceuticals of cell signaling (that is the preparations, that regulate signaling pathways of the stem cells and pathological cells in a mammal organism) in nanocontainers of biodegradable material intact to organelles of stem cells, and to implant nanocontainers loaded with the cell signaling substances into membrane, nucleus or cytoplasm of SC. This provides target delivery of the healthy SC to the site of lesion and activation (launch) of therapeutic inductive cell processes (such as apoptosis, proliferation, differentiation, etc.) immediately in the lesion site, that results from biodegradation of nanocontainers in the required time period thus permitting to avoid undesirable, and frequently harmful action of the mentioned agents of cell signaling onto healthy cells of other body parts.

Set time frame of biodegradation of the nanocontainers allows for the exit of information molecules of cytokines and neurotrophic factors from biologically neutral coating of nanocontainers into SC cytoplasm after the injection of the claimed SC preparation to a patient and provides programmed action of signaling molecules onto requisite components of signaling pathways and/or genes of cell nucleus initiating their expression (induction) and directing development of a cell cycle in predetermined therapeutic way by means of intra- or extracellular signals.

The method of detection of key (controlling) genes is based on fundamental principles of system control theory and on the transcription proteomic analysis of a biopsy sample of pathological site of the patient's neural tissue, culture of his mutant (cancer) SC, isolated from biopsy sample, and native mobilized healthy SC of the patient. When expression of the proteins is significantly lower than the norm the genes are considered key genes. In every specific case the parameters of norm are determined after examination of protein expression of the patient's healthy SC during their transcription proteomic analysis. Quantitative values of protein expression of the key genes of mutant (cancer) SC are basic for the analysis and selection of signaling molecules in order to choose requisite protein or pharmaceutical and calculation of their safe concentration when implanted in the native healthy SC of the patient.

The method of selection of the signaling molecules to reprogram expression (induction) of key genes of autologous SC must be grounded on the following:
1. Signaling molecules must regulate expression (induction) of those signaling systems or key genes of healthy SC which function would enable formation of new intracellular or extracellular signal necessary to initiate the events of specifically programmed cell cycle.
2. In the structure of the signaling pathway of a specific cell event a signaling molecule must ensure the development of cell events according to the preset script at different stages of signal pathways from its release to extracellular response of SC.
3. The signal of a SC extracellular response triggered by intracellular controlling signaling molecule must change biological activity of a cell and orientation of its physiologic functioning.

When choosing signaling preparation of a signaling agent (protein or pharmaceutical) it is feasible to rely on the above mentioned basic principles of transduction reprogramming (expression or induction) key genes of the healthy SC.

Principle of tiered signaling action. The principle consists in the action of extracellular signal of healthy SC onto no less than three various receptors or signaling pathways of the mutant (cancer) SC, thus permitting to double controlling signal at extra- and intracellular levels.

Principle of single vector application of various signals consists in the action of various extracellular signaling molecules of the native SC onto the signaling pathways of mutant (cancer) SC according to rigidly set script of cell cycle events (for example, development of instructive apoptosis).

The principle of dominant controlling signal of the healthy SC implies reprogrammed signal action of the healthy SC onto the mutant (cancer) SC to enhance expression of its inhibited key genes (mostly no less than twice).

As different from the known methods of gene therapy the claimed invention does not set the objective to restore injured genes, to develop mutant genes, steadily producing specific protein, to lyse genes or to arrest translation of the gene. The claimed invention offers "cluster" control of expression (induction) of key genes in specific physiologic orientation of development of cell events According to the invention not only tumor and cancer SC become a target cell, but also a healthy autologous SC transplanted to a patient. The invention demonstrates the opportunity to biologically control SC with interventional programmed action of intra- and extracellular regulation (molecules of signaling proteins) onto the genes of cancer and healthy SC using physiologic mechanisms of cell signaling.

Transcription proteomic comparative analysis of cancer SC and tumor cells with healthy SC of a patient detects key genes and intact parts of pathological cell genome, as well as the disorders of signaling pathways of tumor cell genome [7]. The control for comparison is the values of gene expression for healthy (hematopoietic, neural or mesenchymal) SC. Key genes of cancer SC are defined as the genes with the lowest expression, and the highest values permit verification of the structure of signaling pathways disorders. The analysis of expression of produced key genes of cancer SC and tumor cells permit to detect pathology of the main signaling pathways of the cells of the lesion of brain, to monitor molecular processes of a pathologic cell in the course of the therapy both *in vitro* and *in vivo* and to evaluate efficiency and quality of the administered therapy. Further therapy with the claimed preparation of SC triggers instructive inductive controlled cell processes in pathologic cells (inductive instructive apoptosis, inductive targeted differentiation and commitment of the cells in tumor, inductive programmed proliferation, reprogrammed activation of key genes expression to activate cancer SC in order to enhance efficiency of radiological and chemical therapy etc.). Influence on transduction of genes and signaling pathways of target cells is achieved through short inducing signals of cellular-intercellular interaction from signaling molecules (cytokines, monoclonal antibodies, recombinant ligands, growth factors, proteins of heat shock etc.) released from biodegradable nanocontainers, thus triggering promoters and inhibitors of inhibitors of SC nucleus genes.

The signals go inside the cell and extracellular space in due time after administration of the claimed preparation of SC to the patient. The time is determined by the start of biodegradation of nanocontainers and is necessary for the SC of the claimed preparation to deliver the nanocontainers loaded with the molecules of signaling substance to the site due to their ability to migrate to the site of pathology. Hence tiered signaling action and multiple vector action of influence onto different receptors of the same target cells must be provided in the same therapeutic orientation.

### Brief Description of Drawings

The claimed invention is explicated with the drawings.
Figure 1 shows the scheme of signaling pathways of formation of apoptosis (arrows point to possible places of regulatory signaling action applied to form algorithmized regulation of apoptosis in autologous SC);

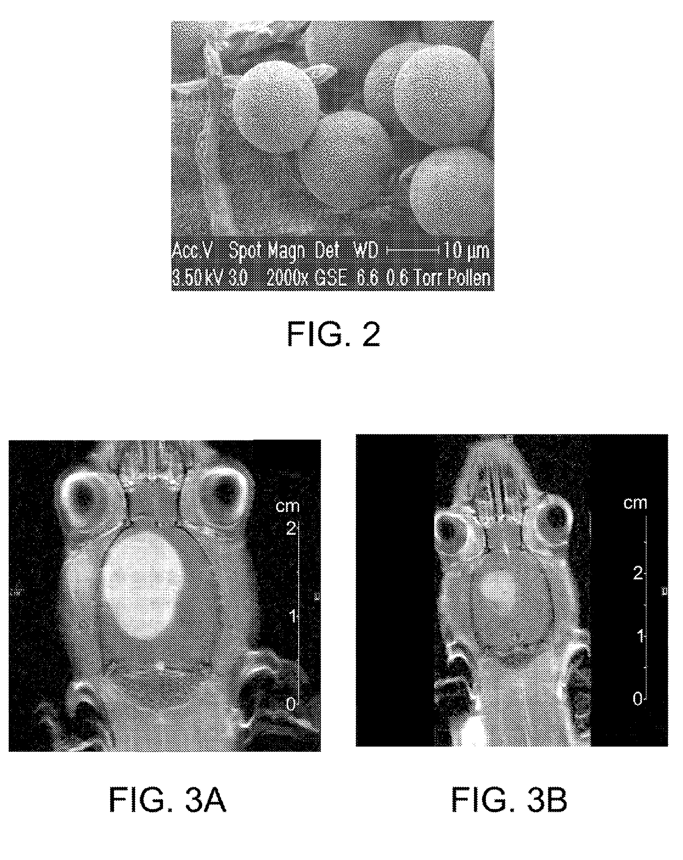

Figure 2 shows electronic microscopy of nanocontainers with protein; nanocontainers are made of biopolymers;
Figure 3 shows MRI of rat brain with experimental C6 glioma 18 days (Fig. 3A) and 26 days (Fig.3B) post implantation of the claimed preparation of SC with nanocontainers with apoptosis promoters (axial projection; T1 weighted image contrasted with Magnevist).

Below are the basic examples of the production of the claimed preparation of SC with reprogrammed cell signaling according to the claimed invention.

### Description of Preferred Embodiments

### Selection of the basic preparation of SC

The basic cell preparation was produced on the basis of the standard preparation of hematopoietic SC, their method of production being described in patent RU 2283119 (MIIK A61K 35/14, .09.2006) for "Preparation of autologous hematopoietic stem cells, their method of production, cryopreservation and application for traumatic disease of central nervous system".

Autologous neural stem cells (NSC) obtained from olfactory sheath of a patient's nose can be used for basic preparation. The method and protocol of NSC production are published [11-14].

The tissue of olfactory sheath including olfactory epithelium and the layer of connective tissue (lamina propria) is obtained from the patients and processed according to standard cultural protocol described in academic literature [15, 16]. Size 10·5mm fragments of mucosa dissected under local anesthesia from the upper part of superior nasal meatus were accepted for research. Sampled tissue was delivered to laboratory in cool Hank's solution without Ca²⁺ and Mg²⁺ (HBSS) containing antibiotic and antimycotic agents (1:100; Gibco). Delivery time was no more than 2 hours. After repeated wash in the same solution blood vessels were removed from mucosa, then the tissue was minced and incubated for 40 minutes at 36.5°C in 0.25% trypsin/EDTA solution prepared on M phosphate buffer (PBS, pH 7.4). Activity of ferments was blocked by DMEM medium (Gibco) that contained 3% of serum, tissue was washed three times in new Hanks' balanced salt solution (HBSS, Sigma) and dissociated by repeated pipetting in nutritive medium. Medium composition: 90% minimum Eagle medium (MEM, Sigma), fetal bovine serum (FBS, Gibco, Invitrogen), 0.8% glucose, 2mM glutamine (Gibco), B27 supplement (Sigma), HEPES 20, growth factors (only for primary cultures), fibroblast growth factor (FGF2, 1ng/ml, Sigma), neural growth factor (NGF 2ng/ml, Sigma).

Received cell suspension was centrifuged (3 minutes at 1200 revolutions per minute), the sediment was resuspended in nutritive medium of the same composition.

The number and viability of cells were checked in Gorjaev's chamber after 0.1% trypan blue staining. Cell suspensions with 85-95% of viable cells were used for further culturing.

Dissociated cells (5·10⁵) cells per ml) were cultured in 12-well trays on polylysine/laminine substratum for 14 days (36.5° C, 5% CO₂). Partial change of 1/3 nutritive medium was done 2 times a week. Primary culture after confluent cell monolayer was removed by trypsin/EDTA solution. After wash in HBSS and centrifuging the cells were resuspended in nutritive medium. Cell suspension (10 000-12 000 cells per cm²) was placed into 12-well trays or dishes (square 25cm²). So the cultures were passed 4 times till confluent monolayer was formed. Free floating and attached to substratum neurospheres formed in cell monolayer were selected by Pastuer pipette and dissociated by the method of ferment processing described above. This permitted separation of neurospheres from accessory glial cells, fibroblasts and stromal (foot) cells. Cell suspension of neurospheres after washing and centrifuging was resuspended in culture medium and cultured in 12-well trays (10000-12000 cells per cm²) and on cover slides (18.18mem) in Petri dishes till confluent monolayer was formed. Received cultures were used for cytological and immunocytochemical tests. Part of the cells of last passages was frozen in cryopreservation medium (90% serum, 10% DMSO) and stored in liquid nitrogen.

Cell monolayer was fixated in 4% solution of paraformaldehyde prepared on 0.01 phosphate buffer (pH 7.4) for 30 minutes. After PBS wash (3·10min) cells were incubated for 24 hours at 4°C with primary antibodies to β-tubulin (1:300; Chemicon), nestin (1:100, Chemicon) and neural specific enolase (1:100, antibodies received in our laboratory). After PBS wash the cells were successively processed by biotinylated antibodies with avidin-biotin complex (ABC, Vector laboratories, Inc), diaminobenzidine solution, prepared on phosphate buffer (DBA 0.5 mg/ml, hydrogen dioxide 0.03%). Preparations were dehydrated and placed into synthetic resin under cover slides (Entellan, Merk).

To avoid graft-versus-host reaction it is necessary to apply only autologous biomaterial, for example NSC isolated from olfactory sheath of the patient's nose or the preparation of hematopoietic stem cells (CD34+) isolated from highly purified mixture of mononuclears isolated from leukoconcentrate of mobilized SC of peripheral blood. The culture of mesenchymal SC isolated from biopsy sample of the patient's bone marrow can also be applied. However, the opportunity to apply basic preparation of allogeneic SC cannot be completely excluded.

### Selection of protein or pharmaceutical

Signaling proteins or pharmaceutical able to regulate signaling pathways of SC and cells of pathologic site are selected individually in each case depending on the objective of inductive cell therapy.

The table below shows the examples of the therapy using available signaling agents (substances that influence cancer SC or its niche) that can be used in the claimed invention.

### Inductive (Signaling) Therapy Targeted to Tumor Cells and Cancer SC

**Table**

| Active agent | Tumor type | Application point | Action mechanism | Result | Authors of the method |
|---|---|---|---|---|---|
| Tykerb | Breast cancer | Protein produced by HER2/neu gene | Eliminates HER2/neu of | CSC percentage in tumour CSC < from 11% to 5% in 63% - recovery. | Jenny Chang Baylor University December 2007 |
| Gleevec | Chronic myeloleucosis. Medulloblastoma | BCR-ABL Signaling pathways | Inhibits BCR-ABL in cancer SC signaling pathways | Block drugs targeting in CSC | Mikle Din National Cancer Institute (USA) 2001 |
| Feverfew extract | Acute myeloleucosis | CSC of acute myeloleucosis | Parthenolide content blocks key enzyme of CSC proliferation | Induces suicide in acute myeloleucosis CSC not involving normal SC | Craig T.Jordan Monica Guzman, Rochester University |
| Rapamycin (Sirolimus, Rapamune) | Leucosis | Pten gene | CSC genes | Suppresses CSC growth and stimulates normal SC growth | Sean Morrison Michigan University 2007 |
| Cyclopamine form Veratrum californicum extract | Prostate cancer Colon cancer Glioblastomas | Wnt and Sonic hedgehog signaling pathways | Inhibits Wnt and Sonic hedgehog signaling pathways | Suppresses CSC proliferation | Michael Din, 2007 National Cancer Institute |
| Monoclonal antibodies to CD44 protein | Myeloid leukemia | CD44 Membrane marker of CSC | Blocks CD44 protein on CSC surface | Immobolizes CSC blocking migration pathways of CSC to the niches. | Princess Margaret Hospital in Toronto(Canada), 2007 |
| Monoclonal antibodies to CD133+ protein | Colon cancer | CSC membrane marker | CD133+ Cells grow as tumor spheres | Block stem factor of CSC | John Dick et.al 2006 (Canada) Ruggero De Maria et al, 2006 (Italy) |
| Adeniviral vector carrying gene interferon b | Glioma | Production of interferon b | Locally create high level of interferon - b in glioma | Antiproliferative and proapoptotic influence towards CSC | Nacamizo A. et al., 2005 |

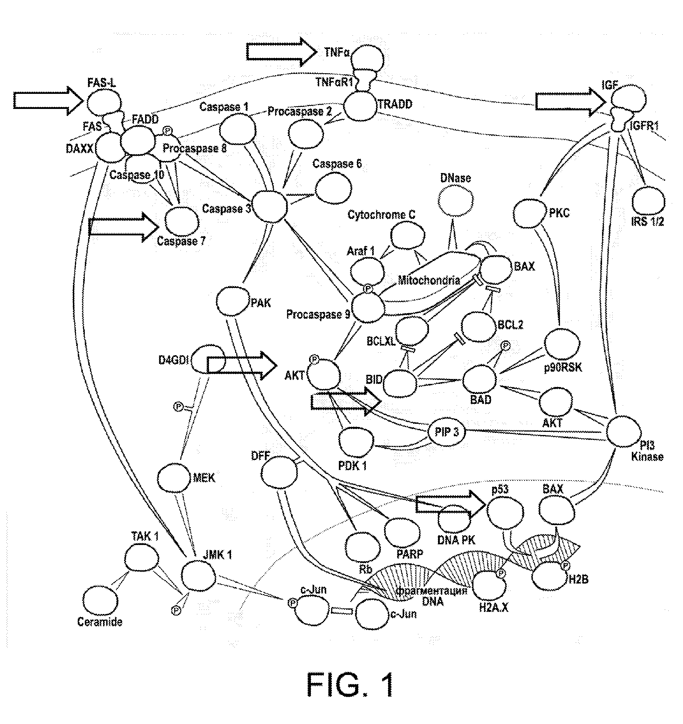

Considering that apoptosis is the best examined cell process, it was chosen to study new cell preparation with reprogrammable properties on the model of apoptosis. The general scheme of signaling pathways of the formation of apoptosis is shown at Figure 1. The scheme demonstrates that apoptosis processes trigger extracellular molecules of signaling proteins of necrosis of factor of tumor (TNFα), proteins of death (FAS-L), insulin growth factor (IGF). The process can be activated on a cell level affecting caspases and p53 gene. Thus, to program inductive instructive apoptosis in brain tumor, the substances participating in signaling chains of apoptosis must be chosen as signaling proteins. Vicin and viscumin can serve as the example of such substances.

The authors of the claimed invention organized laboratory research to examine cytotoxic action of ricin and viscumin on the cells of C6 neuroglioma. These proteins were demonstrated to be functionally active in relation to the cells of this line. LD50 value made 5x10⁻¹² mol for ricin and 5x10⁻¹¹ mol for viscumin. Four specific methods showed that both protein toxins initiated apoptosis of C6 neuroglioma cells. Microscopy tests detected nuclei fragmentation and phosphatydil serine exposition at cell membrane. Flow cytofluorimeter detected the percentage of cells with fragmented DNA being processed with these toxins. Electrophoresis showed specific fragmentation of DNA characteristic for caspase-3-dependant apoptotic endonuclease CAD.

Rapamicin can be mentioned as the example of a pharmaceutical applied in the claimed invention, as the substance to suppress growth of cancer SC and to stimulate growth of normal SC (see the table).

### Encapsulating of protein or pharmaceutical in nanocontainers of biodegradable material

The authors of the claimed invention developed technical specifications for the production of nanocontainers (nanocapsules):
- The size of nanocapsules must not exceed 100 nm.
- Nanocapsules must hermetically contain signaling agent inside and be covered by biodegradable biopolymer or liposomal coating biologically neutral for the body and have preset period of biodegradation inside the body.
- Nanocapsules must be neutral for all internal cell compartments, and easy in production.
- Biodegradation time of nanocapsules must begin at rigidly set time after administration of SC preparation to a patient. Depending on specific conditions and objectives of the therapy the period can vary from one or two hours to several days and months.
- The nanocapsule (nanocontainer) should permit controllable (programmed) biodegradation under the influence of the method of ionizing radiation, laser radiation and/or chemical agents, in order to control cell signaling following prior determined algorithm (program) depending on the exact conditions and objectives of the therapy.
- The nanocapsule must biodegrade into safe components for a cell.
- The nanocapsule (nanocontainer) must be contrast for the methods of radiological diagnostics and be easily diagnosed by computer and magnetic resonance tomography.
- The molecules of signaling substance inside the nanocapsules must fully preserve their potential activity to fully release it after the exit from nanocapsules due to it biodegradation.
- The nanocapsule must be easily implanted through cytoplasm membrane of a cell by endocytosis, by direct intracellular injection or by physiologic approaches or physical-chemical methods.
- In certain cases biodegradable material of nanocapsule coating must be able to conjugate with lipids to provide for the nanocapsules the opportunity to attach to plasmatic membrane of SC.
- The claimed cell preparation with nanocapsules must meet safety criteria and be non-toxic.

To produce the nanocapsules biodegradable material must be properly chosen. Depending on the tasks of the claimed invention nanocapsule of three types have been chosen to deliver signaling protein to target cells:
1) biodegradable nanocapsules produced from biopolymer materials (co-polymers of polylactid glycolid and co-polymers polyhydroxybutirate-valerate);
2) biodegradable nanocapsules made of liposomes;
3) nanoemulsions.

In all cases the size of the obtained nanoparticles varied from 10 to 100nm thus enabling passive diffusion into stem cell or active capture by the cells through pinocytosis, while nanoparticles over 100nm were distributed only in intercellular space.

Obtained nanocapsules with molecules of signaling protein inside were placed in plastic 1.5ml tubes and stored at -12°C till further analysis and use.

### Biodegradable nanocapsules of biopolymer materials (co-polymers of polylactide-glycolide and hydroxybutyrate-valerate)

Signaling protein was encapsulated into biopolymer (polylactide-glycolide or hydroxybutyrate-valerate) carrier according to the following algorithm: weighed portion of lyophilizated signaling protein preliminarily mixed with fine powder of polylactide (average particle size 10-20µm) was loaded into the high pressure chamber with further CO₂ ballooning. The chamber temperature was 50°C, nozzle temperature was about 30°C. The pressure of supercritical CO₂ made 10 MPa. The content of autoclave was thoroughly mixed with magnetic mixer (150 revolutions per minute) for 60 minutes with further output of received mixture and carbon dioxide through the nozzle in suction chamber. After three hours exposure to atmospheric conditions (requisite for complete CO₂ removal and final consolidation of polymer) gathered particles were dispersed and hydrated till average size of nanocapsules did not exceed 10nm.

Ultradispersion was done in T18 basic ULTRA-TURRAX disperser. Dispersing element was S18N-19G (IKA-WERKE GMBH & CO. KG, Germany).

### Liposomic biodegradable nanocapsules

Preferable methods of production of mixed liposomes from non phospholipid amphiphilic membrane mimetics and phospholipids are based on phase behavior of amphiphilic lipids and phospholipids. The ratio of aqueous solution of amphiphilic membrane mimetic and aqueous solution of phospholipid varied from 5:1 to 20:1. Mixing temperature makes to 20PAGE At times amphiphilic membrane mimetic and other components have to be warmed in order to obtain homogeneous aqueous solution before adding phospholipids. Protein or pharmaceutical of signaling agent was added to the ingredients during mixing.

Liposomes of specific size were obtained in two ways:
- The ingredients were added in certain order, intensively mixed till multilamellar vesicles were obtained. Then the particles were minced till single lamellar or small multilamellar liposomes.
- By atomizers: a) injection of phospholipid solution at a high speed through one nozzle into water phase of amphiphilic membrane mimetic; b) injection of amphiphilic membrane mimetic minimum through one nozzle in water phase of phospholipid; c) injection of phospholipid solution at a high speed minimum through one nozzle simultaneously with injection of amphiphilic membrane mimetic solution minimum through one nozzle in a joint box.

### Nanoemulsions

Ultramicroemulsions (mot more than 10µm) were received basing on spontaneously dispersing concentrates of pharmacologically active compound ethers (ACE), such as undecanoates, laureates, palmitates and stearates. Spontaneously dispersing concentrate is diluted by phosphate buffer with signaling protein, and thermodynamically stable microemulsion with 1.5-3µm radius micelles is obtained. Emulsifiers, stabilizators etc. are added to extend life period of "water in oil" emulsion. Signaling proteins are in aqueous phase.

Determining period of biodegradation of nanocapsules in a cell special attention should be given to the type of substance to encapsulate, as well as the method of production of nanocapsules that will permit to program the time of exit of signaling substance from nanocapsule into an autologous SC in rigidly set time.

Biocompatible properties of the proposed nanocapsules are experimentally proved and confirmed.

### Implantation of nanocontainers in SC of the basic preparation

The nanocontainers (nanocapsules) in SC of the basic preparation were implanted by means of exposure of suspension or emulsion of nanocapsules with molecules of signaling protein to the basic preparation of SC for 60 minutes or by culturing of autologous SC with the solution of nanocapsules with signaling protein molecules for 24 hours in standard conditions.

Moreover implantation of nanocapsules according to the claimed invention implies an opportunity to apply standard methods of gene therapy, such as direct intracellular injections of suspension or emulsion of nanocapsules in membrane, cytoplasm or nucleus of SC of basic preparation, as well as use of nanocapsules conjugates with cholera toxin, cholesterol, polyethylen glycol and asialo mucoprotein, use of cationic lipids with nanocapsules, covalent lypophilic chemicals with nanocapsules, hydrocarbon enhancers of cell permeability and membrane fusion gene components.

In certain cases composite nanocapsules were prepared with the coating consisting of biodegradable biopolymer and proteins of the coating of herpetic virus. Use of neurotropic proteins of herpes virus for nanocapsule coating imitate pseudoviral capsid. In this case external coating of a nanocapsule can merge with the plasmatic membrane of an autologous SC, hence nanocapsules with molecules of signaling protein easily penetrate SC by active endocytosis.

The described methods of encapsulating of protein water soluble preparations in nanocontainers can be also used for superselective transfer of conventional chemotherapeutic agents used for cytotoxic and cytoreductive therapy. The claimed preparation of autologous SC with nanocontainers permits target delivery of nanocontainers with pharmaceutics to the site of tumor through migration mechanisms of SC homing to the lesion site.

The preparation of SC with reprogrammed cell signaling obtained according to the claimed invention can be administered to patients by available methods, for example, by intracerebral, intramedullar, intraventricular or intravascular transplantation depending on the objective of the therapy, as well by implantation into tissue-engineering constructions used to fill the defects of brain and spinal cord.

According to the above said the claimed invention can be viewed as new direction in the therapy of tumors, ischemic, degenerative and traumatic injuries of CNS and be qualified as highly technological method of cell cytocorrective or cytoregulatory therapy of CNS tumors, as well as the therapy of other solid tumors and somatic disorders.

The concrete examples of the application of the claimed invention and the methods used at that are brought in the present description only in explicatory aims and do not limit the range and essence of the invention, determined solely by the invention formula. The experts in the field will understand various versions and modifications of the claimed invention that do not go beyond the limits of the invention formula.

### REFERENCES

1. Bryukhovetskiy A.S. Transplantation of neural cells and tissue engineering of brain in nervous diseases. - Moscow.: ZAO Klinika Neuro Vita, 2003.
2. Kobayashi N., Navarro-Alvares N., Soto-Guetierrez A., Kavamoto Hironobu. et al. Cancer Stem Cells Research: Currently Situation and Problems//Cell Transplantation, 2008, Vol.17, p. 19-25.
3. Bryukhovetskiy A.S., Chekhonin V.P., Mentkevich G.L. Stem cells in neurooncology: healthy and cancer stem cells, their possible role and place in cancerogenesis and modern high-technological scripts of brain tumor therapy. Materials of Research and Practice Conference "High technologies in the therapy and rehabilitation of nervous system disorders" - Moscow, 2008, page 43-44.
4. Introduction into molecular medicine. Editor A.A. Paltsev - Moscow: Medicina, 2006.
5. Tsymbalyuk V.I., Medvedev V.V. Neurogenic stem cells. - Kiev: Koval, 2005.
6. ZigovaT., Snyder E., Sanberg P. Neural Stem Cells for Brain and Spinal Cord Repair, 2003.
7. Paltsev M.A., Ivanov A.A., Severin S.E. Intercellular interactions. - Moscow.: Medicina, 2003.
8. Neimark Y.I., Cogan R.Y., Savelyev V. P. Dynamic models of control theory. - Moscow: Nauka, 1985.
9. Grodins F. Regulation theory and biological systems. - Moscow.: Mir, 1966.
10. Fokin S. V., Berkinblit M.B. Mathematic problems in biology. - Moscow, 1973.
11. Zhang X., Klueber K. M., Guo Z. // Experimental Neurology, - 2004, Vol. 186, p. 112-123.
12. Zhang X., Cai J., Klueber K.M. et al. // Stem Cells, 2005, Vol. 23,-p. 442-453.
13. Zhang X., Cai J., Klueber K. M. at al. // Stem Cells, 2006a, Vol. 24, p. 434-442.
14. Zhang X., Klueber K.M., Guo Z. et al. // Brain Res., 2006b, Vol. 1073-1074, p. 109-119.
15. Marshall C.T., Guo Z., Lu C. et al. // Brain Res., 2005, Vol. 1045, p. 45-56.
16. Marshall C.T., Lu C., Winstead W. et al. // Histol. Histopatol., 2006, Vol. 21, p. 633-643.

## Claims

1. A preparation of stem cells with reprogrammed cell signaling, comprising a basic preparation of stem cells, the membrane and/or nucleus, and/or cytoplasm of which has implanted a protein or a pharmaceutical preparation able to regulate signaling pathways of stem cells and cells of a pathological area in a mammal and previously encapsulated into nanocontainers produced from a biodegradable material intact for organelles and compartments of the basic preparation stem cells and having preset period of biodegradation in the mammal to provide programmed exit of the protein or pharmaceutical preparation in intra- or intercellular space of the stem cells and thus reprogramming signaling transduction of key genes of the stem cells in a necessary direction of the development of cell cycle events directly in the pathologic site or an organ or tissue of an organism.

2. The preparation of claim 1 wherein the stem cells of the basic preparation are autologous stem cells.

3. The preparation of claim 1 wherein the size of the nanocontainers does not exceed 100 nm.

4. The preparation of claim 1 wherein the nanocontainers are nanocontainers based on biopolymer materials.

5. The preparation of claim 4 wherein co-polymers polylactide glycolide and/or polyhydroxybutirate-valerate are used as the biopolymer material.

6. The preparation of claim 1 wherein the nanocontainers are nanocontainers based on liposomes.

7. The preparation of claim 1 wherein the nanocontainers are nanoemulsions.

8. A method of producing a preparation of stem cells with reprogrammed signaling, comprising:
selecting a basic preparation of the stem cells,
selecting a protein or pharmaceutical preparation able to regulate signaling pathways of stem cells and cells of a pathological area in a mammal,
encapsulating the selected protein or pharmaceutical preparation in nanocontainers of a biodegradable material intact for organelles and compartments of the stem cells of the basic preparation and having set period of biodegradation in the mammal, and
implanting the nanocontainers in the membrane and/or nucleus, and/or cytoplasm of the stem cells of the basic preparation.

9. The method of claim 8 wherein the basic preparation comprises autologous stem cells.

10. The method of claim 8 wherein encapsulation is carried out by ultradispersion of a mixture of the biopolymer material with the protein or pharmaceutical preparation.

11. The method of claim 8 wherein the nanocontainers are implanted by direct intracellular injections of a suspension or emulsion of the nanocontainers in the membrane, cytoplasm or nucleus of the stem cells of the basic preparation.

12. The method of claim 8 wherein the nanocontainers are implanted by endocytosis in the cytoplasm of the stem cells when suspension or emulsion of nanocontainers is exposed to the basic preparation.

13. The method of claim 8 wherein it is performed under sterile conditions, directly in a operation room (ex temporo) or in a culture laboratory.

14. Use of the preparation of the stem cells according to any of claims 1 to 7 as means of intra- and extra-cellular regulation of signaling pathways of pathological cells in therapy of diseases, injuries and tumors of brain and spinal cord as well as tumors and metastatic lesions of other organs and systems of mammals.
